# EUROPEAN PATENT APPLICATION

(11) **EP 4 509 087 A1**
(43) Date of publication of application: **19.02.2025**
(21) Application number: 24194467.7
(22) Date of filing: 14.08.2024
(51) Int. Cl.: A61B 34/37, A61B 1/313, A61B 34/00, A61B 90/00

(54) **SURGICAL ROBOTIC SYSTEM AND METHOD FOR INPUT SCALING COMPENSATION FOR TELEOPERATIVE LATENCY**

(30) Priority: 15.08.2023 US 202363519614 P; 09.07.2024 US 202418766859
(71) Applicant: Covidien LP, Mansfield, MA 02048 (US)
(72) Inventor: PIAS, Matthew S., 02139 Cambridge (US); ELMAANAOUI, Badr, 02210 Boston (US)
(74) Representative: Maschio & Soames IP Ltd

(57) **Abstract**

A surgical robotic system includes a surgical robotic arm having an instrument and an instrument drive unit actuating the instrument. The system also includes a surgeon console having a handle controller receiving user motion input to control one of the surgical robotic arm or the instrument using a motion scaling factor. The system also includes a laparoscopic camera capturing a video feed and a controller determining a latency attributable to at least one of the video feed or the user motion input and adjusting the motion scaling factor to compensate for the latency.

## Description

### CROSS-REFERENCE TO RELATED APPLICATIONS

This application claims the benefit of, and priority to, U.S. Provisional Patent Application Serial No. 63/519,614 filed on August 15, 2023. The entire contents of the foregoing application is incorporated by reference herein.

### BACKGROUND

Surgical robotic systems are currently being used in a variety of surgical procedures, including minimally invasive surgical procedures. Some surgical robotic systems include a surgeon console providing for teleoperative control of a surgical robotic arm and a surgical instrument having an end effector (e.g., forceps or grasping instrument) coupled to and actuated by the robotic arm. In operation, the robotic arm is moved to a position over a patient and then guides the surgical instrument into a small incision via a surgical port or a natural orifice of a patient to position the end effector at a work site within the patient's body.

A laparoscopic camera, which is also held by one of the robotic arms, is inserted into the patient to image the surgical site. Thus, the teleoperation of the surgical robotic system utilizes two key pipelines: 1) the instrument camera control pipeline, generalized as input, and 2) the video display providing endoscopic video and other visual information, generalized as output. Both pipelines are subject to delay or lag in information delivery, manifesting as pipeline latency. On the input side, the delay between user input at the control device and the corresponding output of instrument or endoscope movement defines input latency. On the output side, the delay between when an event occurs and is captured by the camera and when this event is displayed on the output monitor visible to the user defines output latency.

In many cases, input latency and output latency are indiscernible from each other from the teleoperative user's perspective, and that higher latency correlates with poor usability. A wide range of user experience features (e.g., visual enhancements) potentially impact input and/or output latency or in some cases both. Near infra-red (NIR) imaging results in increased latency across both pipelines reaching an acceptable operating threshold, e.g., 80 milliseconds (ms), as a side effect of introducing a visualization feature offering fluorescence overlay on the laparoscopic camera feed. Additional visualization modes may potentially increase the total latency even further, making the system less usable or potentially even exceeding the acceptable threshold for latency, e.g., remote system operation, where the teleoperative user operates the system from a remote location, introducing network latency in addition to the existing system latency.

While latency may be lessened by improvements to system design (e.g., faster processors, more efficient algorithm design, more efficient instrument and interface mechanical design, etc.) there is a need to compensate for system latency without extensive hardware redesigns.

### SUMMARY

The present disclosure provides the ability to change parameters related to teleoperative input and output to compensate for latency. Specifically, the instrument motion scaling factor is adjusted as a function of system latency to mitigate impacts to system usability because of elevated latency.

The system includes a surgeon console including a pair of handle controllers for moving and actuating one or more robotic arms holding a surgical instrument or a laparoscopic camera. The system also includes sensors for measuring user input, e.g., at the handle controllers, and the resulting compound output of the instrument or the camera. The resulting delay is calculated between the measurement of these outputs by controller of the surgical system. Alternatively, the electrical signal transform of the user input can be compared with the motor output of the instruments/camera to calculate latency.

Measurement of the visualization latency can be done by sending a specific signal having a specific frequency, pattern, etc. of light via a laparoscopic light source or via an external light source and then detecting the arrival time of that signal of light via a sensor located on the output monitor or the laparoscopic camera imaging sensor. The delay in the light input and monitor sensor measurement can be calculated by one or more processors of the surgical robotic system.

In embodiments, latency measurement may be done during manufacture. A fixed motion scaling factor can be set for the system, scaling the manipulation of instrument and/or camera output as related to the input per a predetermined formula. In further embodiments, a range of motion scaling factors can be made available to the user that can be adjusted during device operation. If measurement of latency is done on system, a scaling factor can be introduced either automatically or manually. Latency measurement may also be triggered automatically or manually by the user. When done automatically, the measurement of latency can occur automatically either at set intervals or as triggered by system events. After automatic or manual measurement occurs, the user may be presented with an option to set a recommended motion scaling factor from a set or range of factors.

Development of new features for the surgical robotic systems may increase system latency as a cost of feature addition. Increased latency demonstrably reduces system usability. Reducing latency can limit the feature scope, increase costs, increase project timeline, etc. The disclosed latency compensation via motion scaling factor adjustment will allow implementation of latency heavy system features.

According to one embodiment of the present disclosure, a surgical robotic system is disclosed. The system includes a surgical robotic arm having an instrument and an instrument drive unit actuating the instrument. The system also includes a surgeon console having a handle controller receiving user motion input to control one of the surgical robotic arm or the instrument using a motion scaling factor. The system also includes a laparoscopic camera capturing a video feed and a controller determining a latency attributable to at least one of the video feed or the user motion input and adjusting the motion scaling factor to compensate for the latency.

Implementations of the above embodiment may include one or more of the following features. According to one aspect of the above embodiment, the controller may determine the latency by determining imaging latency of the video feed. The surgical robotic system may further include a video processing device coupled to the laparoscopic camera for receiving and processing the video feed. The surgical robotic system may further include a screen disposed at the surgeon console and displaying the video feed. The imaging latency may include a delay between capturing of the video feed by the laparoscopic camera and displaying the video feed on the screen. The controller may also determine the latency by determining control latency of the user input. The control latency may include a delay between receiving the user input and actuating one of the surgical robotic arm or the instrument as well as the user adjusting endoscope position and parameters, which are also subject to latency. The controller may further compare the determined latency to a threshold latency and select an adjusted motion scaling factor in response to the determined latency exceeding the threshold latency. In response to the determined latency exceeding the threshold latency, the controller may adjust the motion scaling factor automatically. In response to the determined latency exceeding the threshold latency, the controller may output a prompt on a graphical user interface indicating the determined latency exceeds the threshold latency. The controller may activate latency compensation by adjusting the motion scaling factor in response to a user input to the prompt.

According to a further embodiment of the present disclosure, a method for controlling a surgical robotic system is disclosed. The method includes receiving user motion input at a handle controller of a surgeon console for controlling one of a surgical robotic arm or an instrument using a motion scaling factor. The method also includes capturing a video feed of a surgical site through a laparoscopic camera and determining a latency attributable to one of the video feed or the user motion input. The method further includes adjusting the motion scaling factor to compensate for the latency.

Implementations of the above embodiment may include one or more of the following features. According to one aspect of the above embodiment, determining the latency may further include determining imaging latency of the video feed. The method may also include receiving and processing the video feed at a video processing device coupled to the laparoscopic camera and displaying the video feed at a screen disposed at the surgeon console. The imaging latency may include a delay between capturing of the video feed by the laparoscopic camera and displaying the video feed on the screen. Determining the latency may further include determining control latency of the user input. The control latency may include a delay between receiving the user input and actuating at least one of the surgical robotic arm or the instrument. The method may include comparing the determined latency to a threshold latency and selecting an adjusted motion scaling factor in response to the determined latency exceeding the threshold latency. The method may further include outputting a prompt on a graphical user interface indicating the determined latency exceeds the threshold latency in response to the determined latency exceeding the threshold latency the controller and activating latency compensation by adjusting the motion scaling factor in response to a user input to the prompt.

According to another embodiment of the present disclosure, a surgical robotic system is disclosed. The surgical robotic system includes a surgical robotic arm having an instrument and an instrument drive unit actuating the instrument. The system also includes a storage device storing a range of baseline motion scaling factors based on a latency calibration. The system further includes a surgeon console having a handle controller receiving user motion input to control one of the surgical robotic arm or the instrument using a motion scaling factor selected from the baseline motion scaling factor range. The system further includes a laparoscopic camera capturing a video feed and a controller determining a latency attributable to of the video feed or the user motion input and adjusting the motion scaling factor to compensate for the latency.

Implementations of the above embodiment may include one or more of the following features. According to one aspect of the above embodiment, the controller may compare the determined latency to a threshold latency and select an adjusted motion scaling factor in response to the determined latency exceeding the threshold latency.

### BRIEF DESCRIPTION OF THE DRAWINGS

Various embodiments of the present disclosure are described herein with reference to the drawings wherein:
FIG. 1 is a perspective view of a surgical robotic system including a control tower, a console, and one or more surgical robotic arms each disposed on a mobile cart according to an embodiment of the present disclosure;
FIG. 2 is a perspective view of a surgical robotic arm of the surgical robotic system of FIG. 1 according to an embodiment of the present disclosure;
FIG. 3 is a perspective view of a mobile cart having a setup arm with the surgical robotic arm of the surgical robotic system of FIG. 1 according to an embodiment of the present disclosure;
FIG. 4 is a schematic diagram of a computer architecture of the surgical robotic system of FIG. 1 according to an embodiment of the present disclosure;
FIG. 5 is a plan schematic view of the surgical robotic system of FIG. 1 positioned about a surgical table according to an embodiment of the present disclosure;
FIG. 6 is a schematic diagram of a system for determining phases of a surgical procedure according to an embodiment of the present disclosure;
FIG. 7 is a flow chart of a method for input scaling compensation for teleoperative latency of the surgical robotic system of FIG. 1 according to an embodiment of the present disclosure.

### DETAILED DESCRIPTION

Embodiments of the presently disclosed surgical robotic system are described in detail with reference to the drawings, in which like reference numerals designate identical or corresponding elements in each of the several views. As used herein the term "coupled to" denotes a connection between components, which may be direct or indirect(i.e., through one or more components) and may be electronic, electrical, mechanical, or combinations thereof.

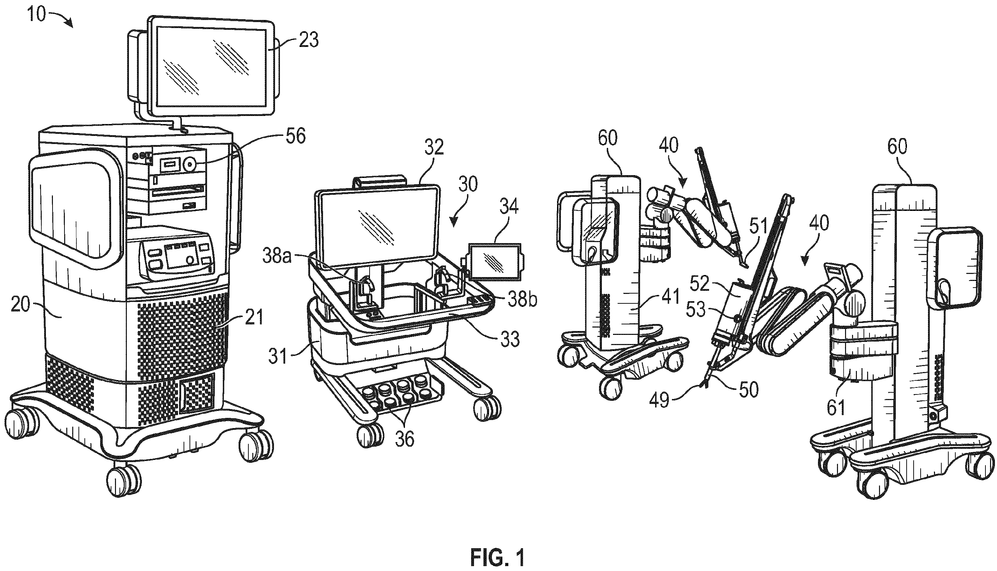

With reference to FIG. 1, a surgical robotic system 10 includes a control tower 20, which is connected to all the components of the surgical robotic system 10 including a surgeon console 30 and one or more mobile carts 60. Each of the mobile carts 60 includes a robotic arm 40 having a surgical instrument 50 removably coupled thereto. The robotic arms 40 also couple to the mobile carts 60. The robotic system 10 may include any number of mobile carts 60 and/or robotic arms 40.

The surgical instrument 50 is configured for use during minimally invasive surgical procedures. In embodiments, the surgical instrument 50 may be configured for open surgical procedures. In further embodiments, the surgical instrument 50 may be an electrosurgical or ultrasonic instrument, such as a forceps configured to seal tissue by compressing tissue between jaw members and applying electrosurgical current or ultrasonic vibrations via an ultrasonic transducer to the tissue. In yet further embodiments, the surgical instrument 50 may be a surgical stapler including a pair of jaws configured to grasp and clamp tissue while deploying a plurality of tissue fasteners, e.g., staples, and cutting stapled tissue. In yet further embodiments, the surgical instrument 50 may be a surgical clip applier including a pair of jaws configured apply a surgical clip onto tissue. The system also includes an electrosurgical generator configured to output electrosurgical (e.g., monopolar or bipolar) or ultrasonic energy in a variety of operating modes, such as coagulation, cutting, sealing, etc. Suitable generators include a Valleylab^{™} FT10 Energy Platform available from Medtronic of Minneapolis, MN.

One of the robotic arms 40 may include a laparoscopic camera 51 configured to capture video of the surgical site. The laparoscopic camera 51 may be a stereoscopic camera configured to capture two side-by-side (i.e., left and right) images of the surgical site to produce a video stream of the surgical scene. The laparoscopic camera 51 is coupled to an image processing device 56, which may be disposed within the control tower 20. The image processing device 56 may be any computing device configured to receive the video feed from the laparoscopic camera 51 and output the processed video stream.

The surgeon console 30 includes a first, i.e., surgeon, screen 32, which displays a video feed of the surgical site provided by camera 51 of the surgical instrument 50 disposed on the robotic arm 40, and a second screen 34, which displays a user interface for controlling the surgical robotic system 10. The first screen 32 and second screen 34 may be touchscreens allowing for displaying various graphical user inputs.

The surgeon console 30 also includes a plurality of user interface devices, such as foot pedals 36 and a pair of hand controllers 38a and 38b which are used by a user to remotely control robotic arms 40. The surgeon console further includes an armrest 33 used to support clinician's arms while operating the hand controllers 38a and 38b.

The control tower 20 includes a screen 23, which may be a touchscreen, and outputs on the graphical user interfaces (GUIs). The control tower 20 also acts as an interface between the surgeon console 30 and one or more robotic arms 40. In particular, the control tower 20 is configured to control the robotic arms 40, such as to move the robotic arms 40 and the corresponding surgical instrument 50, based on a set of programmable instructions and/or input commands from the surgeon console 30, in such a way that robotic arms 40 and the surgical instrument 50 execute a desired movement sequence in response to input from the foot pedals 36 and the hand controllers 38a and 38b. The foot pedals 36 may be used to enable and lock the hand controllers 38a and 38b, repositioning camera movement and electrosurgical activation/deactivation. In particular, the foot pedals 36 may be used to perform a clutching action on the hand controllers 38a and 38b. Clutching is initiated by pressing one of the foot pedals 36, which disconnects (i.e., prevents movement inputs) the hand controllers 38a and/or 38b from the robotic arm 40 and corresponding instrument 50 or camera 51 attached thereto. This allows the user to reposition the hand controllers 38a and 38b without moving the robotic arm(s) 40 and the instrument 50 and/or camera 51. This is useful when reaching control boundaries of the surgical space.

Each of the control tower 20, the surgeon console 30, and the robotic arm 40 includes a respective computer 21, 31, 41. The computers 21, 31, 41 are interconnected to each other using any suitable communication network based on wired or wireless communication protocols. The term "network," whether plural or singular, as used herein, denotes a data network, including, but not limited to, the Internet, Intranet, a wide area network, or a local area network, and without limitation as to the full scope of the definition of communication networks as encompassed by the present disclosure. Suitable protocols include, but are not limited to, transmission control protocol/internet protocol (TCP/IP), datagram protocol/internet protocol (UDP/IP), and/or datagram congestion control protocol (DC). Wireless communication may be achieved via one or more wireless configurations, e.g., radio frequency, optical, Wi-Fi, Bluetooth (an open wireless protocol for exchanging data over short distances, using short length radio waves, from fixed and mobile devices, creating personal area networks (PANs), ZigBee^{®} (a specification for a suite of high level communication protocols using small, low-power digital radios based on the IEEE 122.15.4-1203 standard for wireless personal area networks (WPANs)).

The computers 21, 31, 41 may include any suitable processor (not shown) operably connected to a memory (not shown), which may include one or more of volatile, non-volatile, magnetic, optical, or electrical media, such as read-only memory (ROM), random access memory (RAM), electrically-erasable programmable ROM (EEPROM), non-volatile RAM (NVRAM), or flash memory. The processor may be any suitable processor (e.g., control circuit) adapted to perform the operations, calculations, and/or set of instructions described in the present disclosure including, but not limited to, a hardware processor, a field programmable gate array (FPGA), a digital signal processor (DSP), a central processing unit (CPU), a microprocessor, and combinations thereof. Those skilled in the art will appreciate that the processor may be substituted for by using any logic processor (e.g., control circuit) adapted to execute algorithms, calculations, and/or set of instructions described herein.

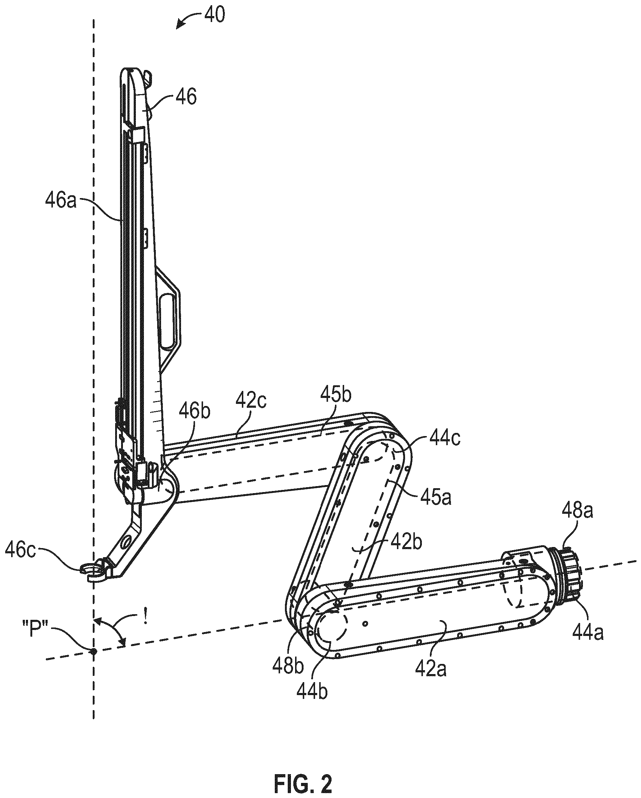

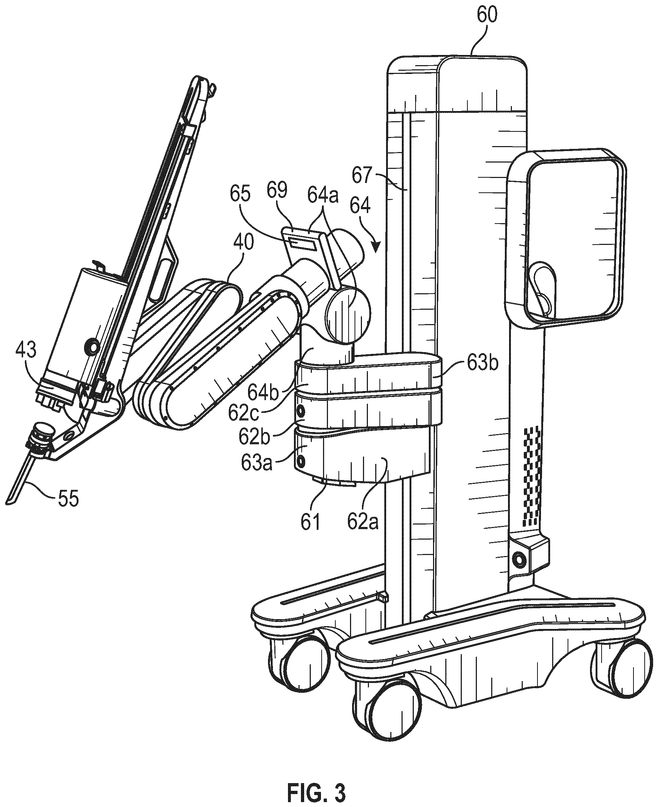

With reference to FIG. 2, each of the robotic arms 40 may include a plurality of links 42a, 42b, 42c, which are interconnected at joints 44a, 44b, 44c, respectively. Other configurations of links and joints may be utilized as known by those skilled in the art. The joint 44a is configured to secure the robotic arm 40 to the mobile cart 60 and defines a first longitudinal axis. With reference to FIG. 3, the mobile cart 60 includes a lift 67 and a setup arm 61, which provides a base for mounting the robotic arm 40. The lift 67 allows for vertical movement of the setup arm 61. The mobile cart 60 also includes a screen 69 for displaying information pertaining to the robotic arm 40. In embodiments, the robotic arm 40 may include any type and/or number of joints.

The setup arm 61 includes a first link 62a, a second link 62b, and a third link 62c, which provide for lateral maneuverability of the robotic arm 40. The links 62a, 62b, 62c are interconnected at joints 63a and 63b, each of which may include an actuator (not shown) for rotating the links 62b and 62b relative to each other and the link 62c. In particular, the links 62a, 62b, 62c are movable in their corresponding lateral planes that are parallel to each other, thereby allowing for extension of the robotic arm 40 relative to the patient (e.g., surgical table). In embodiments, the robotic arm 40 may be coupled to the surgical table (not shown). The setup arm 61 includes controls 65 for adjusting movement of the links 62a, 62b, 62c as well as the lift 67. In embodiments, the setup arm 61 may include any type and/or number of joints.

The third link 62c may include a rotatable base 64 having two degrees of freedom. In particular, the rotatable base 64 includes a first actuator 64a and a second actuator 64b. The first actuator 64a is rotatable about a first stationary arm axis which is perpendicular to a plane defined by the third link 62c and the second actuator 64b is rotatable about a second stationary arm axis which is transverse to the first stationary arm axis. The first and second actuators 64a and 64b allow for full three-dimensional orientation of the robotic arm 40.

The actuator 48b of the joint 44b is coupled to the joint 44c via the belt 45a, and the joint 44c is in turn coupled to the joint 46b via the belt 45b. Joint 44c may include a transfer case coupling the belts 45a and 45b, such that the actuator 48b is configured to rotate each of the links 42b, 42c and a holder 46 relative to each other. More specifically, links 42b, 42c, and the holder 46 are passively coupled to the actuator 48b which enforces rotation about a pivot point "P" which lies at an intersection of the first axis defined by the link 42a and the second axis defined by the holder 46. In other words, the pivot point "P" is a remote center of motion (RCM) for the robotic arm 40. Thus, the actuator 48b controls the angle θ between the first and second axes allowing for orientation of the surgical instrument 50. Due to the interlinking of the links 42a, 42b, 42c, and the holder 46 via the belts 45a and 45b, the angles between the links 42a, 42b, 42c, and the holder 46 are also adjusted to achieve the desired angle θ. In embodiments, some or all of the joints 44a, 44b, 44c may include an actuator to obviate the need for mechanical linkages.

The joints 44a and 44b include an actuator 48a and 48b configured to drive the joints 44a, 44b, 44c relative to each other through a series of belts 45a and 45b or other mechanical linkages such as a drive rod, a cable, or a lever and the like. In particular, the actuator 48a is configured to rotate the robotic arm 40 about a longitudinal axis defined by the link 42a.

With reference to FIG. 2, the holder 46 defines a second longitudinal axis and configured to receive an instrument drive unit (IDU) 52 (FIG. 1). The IDU 52 is configured to couple to an actuation mechanism of the surgical instrument 50 and the camera 51 and is configured to move (e.g., rotate) and actuate the instrument 50 and/or the camera 51. IDU 52 transfers actuation forces from its actuators to the surgical instrument 50 to actuate components an end effector 49 of the surgical instrument 50. The holder 46 includes a sliding mechanism 46a, which is configured to move the IDU 52 along the second longitudinal axis defined by the holder 46. The holder 46 also includes a joint 46b, which rotates the holder 46 relative to the link 42c. During laparoscopic procedures, the instrument 50 may be inserted through a laparoscopic access port 55 (FIG. 3) held by the holder 46. The holder 46 also includes a port latch 46c for securing the access port 55 to the holder 46 (FIG. 2).

The robotic arm 40 also includes a plurality of manual override buttons 53 (FIG. 1) disposed on the IDU 52 and the setup arm 61, which may be used in a manual mode. The user may press one or more of the buttons 53 to move the component associated with the button 53.

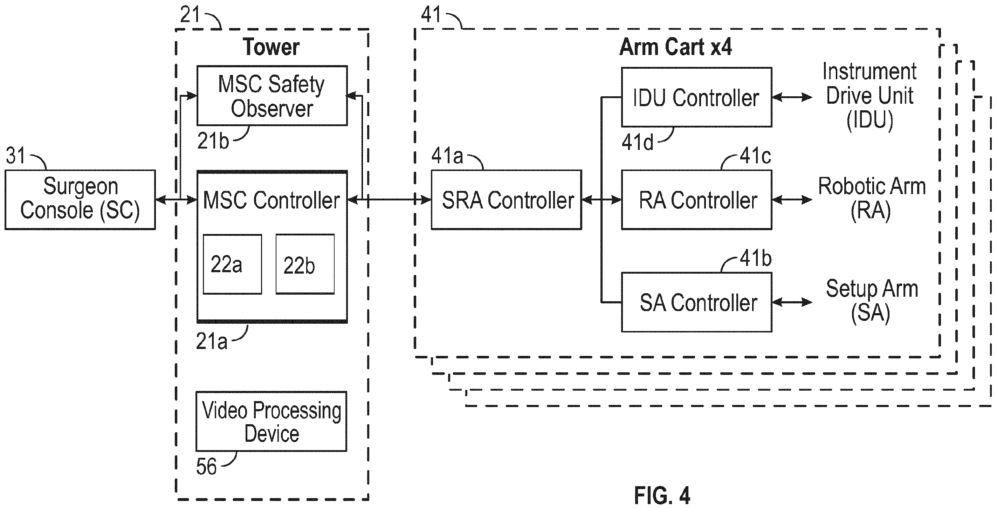

With reference to FIG. 4, each of the computers 21, 31, 41 of the surgical robotic system 10 may include a plurality of controllers, which may be embodied in hardware and/or software. The computer 21 of the control tower 20 includes a controller 21a and safety observer 21b. The controller 21a receives data from the computer 31 of the surgeon console 30 about the current position and/or orientation of the hand controllers 38a and 38b and the state of the foot pedals 36 and other buttons. The controller 21a processes these input positions to determine desired drive commands for each joint of the robotic arm 40 and/or the IDU 52 and communicates these to the computer 41 of the robotic arm 40. The controller 21a also receives the actual joint angles measured by encoders of the actuators 48a and 48b and uses this information to determine force feedback commands that are transmitted back to the computer 31 of the surgeon console 30 to provide haptic feedback through the hand controllers 38a and 38b. The safety observer 21b performs validity checks on the data going into and out of the controller 21a and notifies a system fault handler if errors in the data transmission are detected to place the computer 21 and/or the surgical robotic system 10 into a safe state.

The controller 21a is coupled to a storage 22a, which may be non-transitory computer-readable medium configured to store any suitable computer data, such as software instructions executable by the controller 21a. The controller 21a also includes transitory memory 22b for loading instructions and other computer readable data during execution of the instructions. In embodiments, other controllers of the system 10 include similar configurations.

The computer 41 includes a plurality of controllers, namely, a main cart controller 41a, a setup arm controller 41b, a robotic arm controller 41c, and an instrument drive unit (IDU) controller 41d. The main cart controller 41a receives and processes joint commands from the controller 21a of the computer 21 and communicates them to the setup arm controller 41b, the robotic arm controller 41c, and the IDU controller 41d. The main cart controller 41a also manages instrument exchanges and the overall state of the mobile cart 60, the robotic arm 40, and the IDU 52. The main cart controller 41a also communicates actual joint angles back to the controller 21a.

Each of joints 63a and 63b and the rotatable base 64 of the setup arm 61 are passive joints (i.e., no actuators are present therein) allowing for manual adjustment thereof by a user. The joints 63a and 63b and the rotatable base 64 include brakes that are disengaged by the user to configure the setup arm 61. The setup arm controller 41b monitors slippage of each of joints 63a and 63b and the rotatable base 64 of the setup arm 61, when brakes are engaged or can be freely moved by the operator when brakes are disengaged, but do not impact controls of other joints. The robotic arm controller 41c controls each joint 44a and 44b of the robotic arm 40 and calculates desired motor torques required for gravity compensation, friction compensation, and closed loop position control of the robotic arm 40. The robotic arm controller 41c calculates a movement command based on the calculated torque. The calculated motor commands are then communicated to one or more of the actuators 48a and 48b in the robotic arm 40. The actual joint positions are then transmitted by the actuators 48a and 48b back to the robotic arm controller 41c.

The IDU controller 41d receives desired joint angles for the surgical instrument 50, such as wrist and jaw angles, and computes desired currents for the motors in the IDU 52. The IDU controller 41d calculates actual angles based on the motor positions and transmits the actual angles back to the main cart controller 41a.

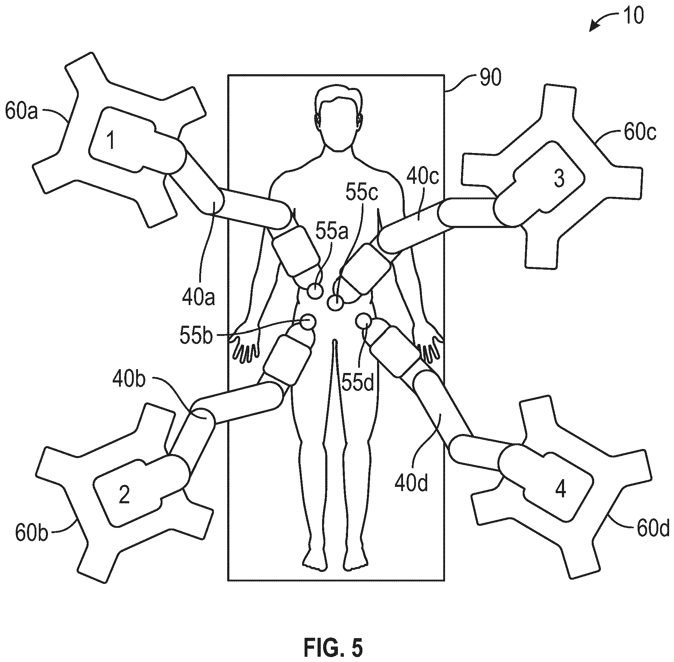

With reference to FIG. 5, the surgical robotic system 10 is set up around a surgical table 90. The system 10 includes mobile carts 60a-d, which may be numbered "1" through "4." During setup, each of the carts 60a-d are positioned around the surgical table 90. Position and orientation of the carts 60a-d depends on a plurality of factors, such as placement of a plurality of access ports 55a-d, which in turn, depends on the surgery being performed. Once the port placement is determined, the access ports 55a-d are inserted into the patient, and carts 60a-d are positioned to insert instruments 50 and the laparoscopic camera 51 into corresponding ports 55a-d.

During use, each of the robotic arms 40a-d is attached to one of the access ports 55a-d that is inserted into the patient by attaching the latch 46c (FIG. 2) to the access port 55 (FIG. 3). The IDU 52 is attached to the holder 46, followed by the SIM 43 being attached to a distal portion of the IDU 52. Thereafter, the instrument 50 is attached to the SIM 43. The instrument 50 is then inserted through the access port 55 by moving the IDU 52 along the holder 46. The SIM 43 includes a plurality of drive shafts configured to transmit rotation of individual motors of the IDU 52 to the instrument 50 thereby actuating the instrument 50. In addition, the SIM 43 provides a sterile barrier between the instrument 50 and the other components of robotic arm 40, including the IDU 52. The SIM 43 is also configured to secure a sterile drape (not shown) to the IDU 52.

A surgical procedure may include multiple phases, and each phase may include one or more surgical actions. As used herein, the term "phase" represents a surgical event that is composed of a series of steps (e.g., closure). A "surgical action" may include an incision, a compression, a stapling, a clipping, a suturing, a cauterization, a sealing, or any other such actions performed to complete a phase in the surgical procedure. A "step" refers to the completion of a named surgical objective (e.g., hemostasis). During each step, certain surgical instruments 50 (e.g., forceps) are used to achieve a specific objective by performing one or more surgical actions.

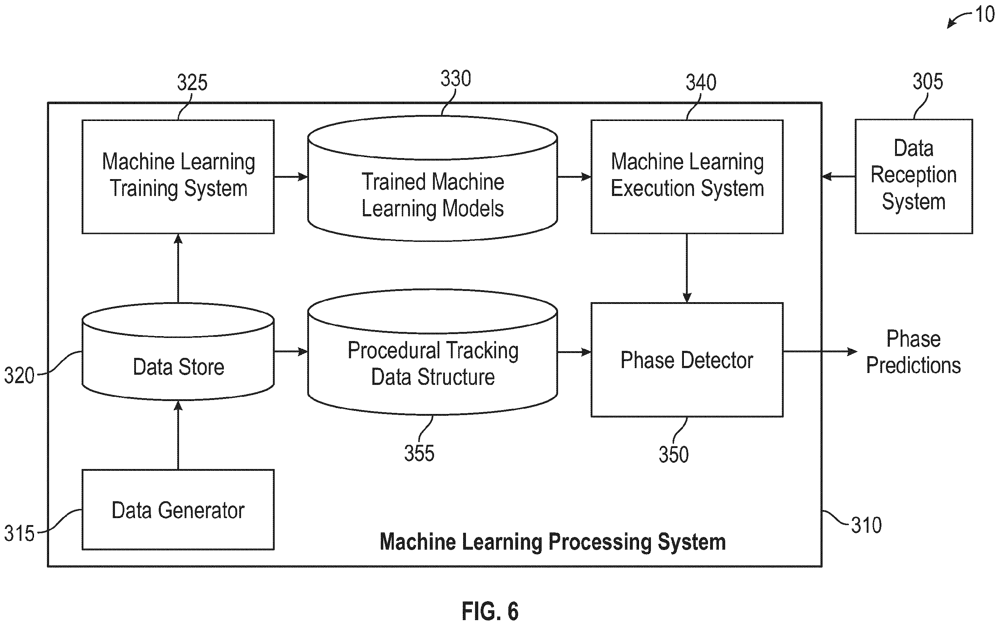

With reference to FIG. 6, the surgical robotic system 10 may include a machine learning (ML) processing system 310 that processes the surgical data using one or more ML models to identify one or more features, such as surgical phase, instrument, anatomical structure, etc., in the surgical data. The ML processing system 310 includes a ML training system 325, which may be a separate device (e.g., server) that stores its output as one or more trained ML models 330. The ML models 330 are accessible by a ML execution system 340. The ML execution system 340 may be separate from the ML training system 325, namely, devices that "train" the models are separate from devices that "infer," i.e., perform real-time processing of surgical data using the trained ML models 330.

System 10 includes a data reception system 305 that collects surgical data, including the video data and surgical instrumentation data. The data reception system 305 can include one or more devices (e.g., one or more user devices and/or servers) located within and/or associated with a surgical operating room and/or control center. The data reception system 305 can receive surgical data in real-time, i.e., as the surgical procedure is being performed.

The ML processing system 310, in some examples, may further include a data generator 315 to generate simulated surgical data, such as a set of virtual images, or record the video data from the image processing device 56, to train the ML models 330 as well as other sources of data, e.g., user input, arm movement, etc. Data generator 315 can access (read/write) a data store 320 to record data, including multiple images and/or multiple videos.

The ML processing system 310 also includes a phase detector 350 that uses the ML models to identify a phase within the surgical procedure. Phase detector 350 uses a particular procedural tracking data structure 355 from a list of procedural tracking data structures. Phase detector 350 selects the procedural tracking data structure 355 based on the type of surgical procedure that is being performed. In one or more examples, the type of surgical procedure is predetermined or input by user. The procedural tracking data structure 355 identifies a set of potential phases that may correspond to a part of the specific type of surgical procedure.

In some examples, the procedural tracking data structure 355 may be a graph that includes a set of nodes and a set of edges, with each node corresponding to a potential phase. The edges may provide directional connections between nodes that indicate (via the direction) an expected order during which the phases will be encountered throughout an iteration of the surgical procedure. The procedural tracking data structure 355 may include one or more branching nodes that feed to multiple next nodes and/or may include one or more points of divergence and/or convergence between the nodes. In some instances, a phase indicates a procedural action (e.g., surgical action) that is being performed or has been performed and/or indicates a combination of actions that have been performed. In some instances, a phase relates to a biological state of a patient undergoing a surgical procedure. For example, the biological state may indicate a complication (e.g., blood clots, clogged arteries/veins, etc.), pre-condition (e.g., lesions, polyps, etc.). In some examples, the ML models 330 are trained to detect an "abnormal condition," such as hemorrhaging, arrhythmias, blood vessel abnormality, etc.

The phase detector 350 outputs the phase prediction associated with a portion of the video data that is analyzed by the ML processing system 310. The phase prediction is associated with the portion of the video data by identifying a start time and an end time of the portion of the video that is analyzed by the ML execution system 340. The phase prediction that is output may include an identity of a surgical phase as detected by the phase detector 350 based on the output of the ML execution system 340. Further, the phase prediction, in one or more examples, may include identities of the structures (e.g., instrument, anatomy, etc.) that are identified by the ML execution system 340 in the portion of the video that is analyzed. The phase prediction may also include a confidence score of the prediction. Other examples may include various other types of information in the phase prediction that is output. The predicted phase may be used by the controller 21a to determine when to switch to measure teleoperative latency and/or switch motion scaling factor as described below.

Motion scaling may be also adjusted based on the detected phase of the surgery. Latency hinders fine movements the most so neutral/positive motion scaling can be automated/suggested for phases with longer/less precise motion and negative motion scaling can be introduced for phases with more precision required. In particular, phase detection can provide indication of types of motion that the surgeon might use (e.g., large sweeping motions vs fine manipulation motions). These can be used as an input to adjust motion scaling. Large sweeping motions by the surgeon could result in the motion scaling adjustment algorithm suggesting/automatically changing the motion scaling factor to be neutral or positive. Small fine movements would result in the algorithm suggesting/automatically changing the motion scaling factor to be negative (scaling down input motion).

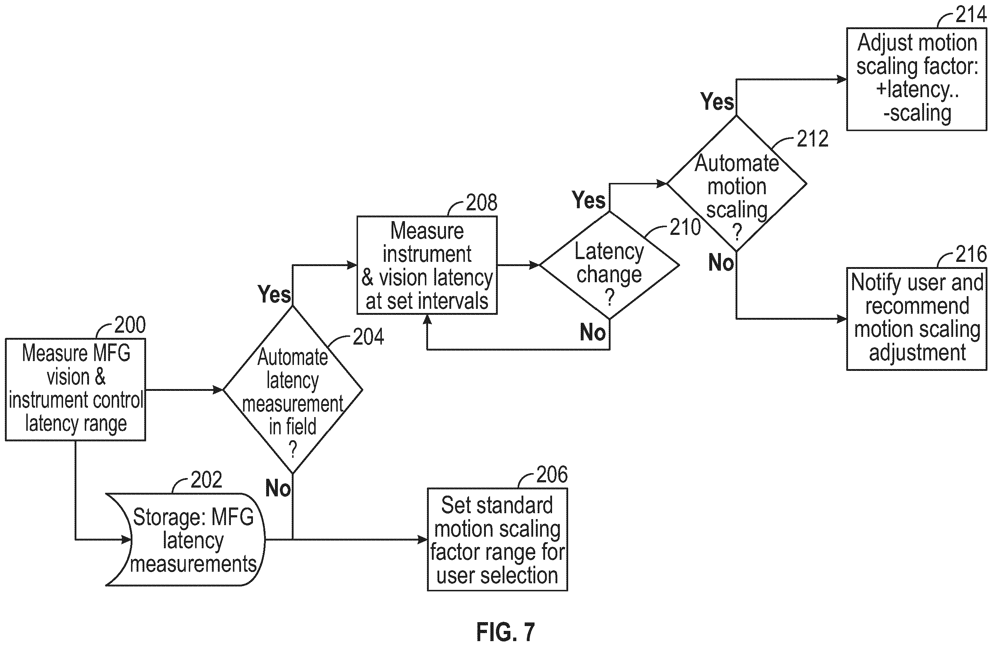

With reference to FIG. 7, a method of input scaling compensation for teleoperative latency of the surgical robotic system of FIG. 1, which includes the image processing device 56 and the camera 51, is shown. The method may be embodied as software instructions stored in non-transitory medium (e.g., memory) of the image processing device 56 and executable by one or more processors (e.g., FPGA, CPU, GPU, etc.) of the image processing device 56 or any of the controllers of the system 10 (e.g., controller 21a).

The camera 51 may be a dual sensor camera capable of imaging white light and NIR imaging using various contrast agents. With intraoperative usage of fluorophores from a fluorescent dye, such as indocyanine green (ICG), the imaging system enables real-time visual assessment and of blood vessels, lymph nodes, lymphatic flow, biliary ducts, and other tissues during surgical procedures. The video processing device 56 is configured to combine the white light and IR images from the camera 51 by displaying reflected NIR light as a visible color (e.g., green, blue, etc.) on the video feed shown on the first screen 32 of the surgeon console 30.

At step 200, latency of the system 10 is measured to determine baseline latency. This may be done during manufacture of the system 10, its deployment, or startup procedure. The latency is measured for the imaging pipeline, which includes camera 51, the image processing device 56, and one or more of the screens 23, 32, 34 displaying the video feed. The imaging pipeline latency refers to delay in transmitting and receiving images.

Measurement of the visualization latency can be done by sending a specific signal having a specific frequency, pattern, etc. of light via a laparoscopic light source of the laparoscopic camera 51 or via an external light source and then detecting the arrival time of that signal of light via a sensor located on the output monitor or the laparoscopic camera imaging sensor. The delay in the light input and monitor sensor measurement can be calculated by the controller 21a of the surgical robotic system 10.

The latency is also measured for the control pipeline, which includes the hand controllers 38a and 38b, the surgeon console 30, the robotic arms 40a-d and whatever devices attached thereto, e.g., instrument 50, camera 51, etc. The control pipeline latency refers to the delay in the robotically controlled device performing the command input through the handle controllers 38a and 38b. In embodiments, latency of one or both of the pipelines may be measured and further processed. The baseline latency may be expressed as unit of time, e.g., milliseconds (ms).

The system also includes sensors for measuring user input, e.g., at the handle controllers 38a and 38b, and the resulting compound output of the robotic arms 40a-d, instrument 50 or the camera 51. The resulting delay is calculated between the measurement of these outputs by controller 21a of the surgical system 10. Alternatively, the electrical signal transform of the user input can be compared with the motor output of the instruments/camera to calculate control latency.

At step 202, the baseline control and imaging latency values are stored in the storage 22a and are used to adjust motion scaling used in controlling the robotic arms 40a-d and their corresponding instruments 50 and/or camera 51. At step 204, the controller 21a determines whether to automate latency measurement during operation of the system 10, i.e., during surgery. The determination is based on user selection or a system setting. If the latency measurement is not automated, then at step 206 the controller 21a sets the standard motion scaling factor based on the stored baseline latency values. The scaling factor may be loaded as a range of values, allows for selection of a suitable motion scaling factor value based on the measured latency.

If the latency measurement is automated, as determined by the controller 21a at step 204, then the controller 21a measures control and/or imaging latency at step 208 in the same manner as described above at step 200. In embodiments, the currently measured latency may be continuously displayed on the screens 32 and/or 34.

At step 210, the controller 21a determines whether the latency is larger than a latency threshold. Each of the pipeline latencies may have a corresponding latency threshold, e.g., 150 ms for imaging latency and 100 ms for control latency. The controller 21a compares the measured latency to corresponding thresholds, and if the measured latency is below the preset threshold, then the controller 21a continues to measure the latency.

If either of the imaging or control latency is above their corresponding threshold, then at step 212 the controller 21a determines whether the motion scaling is automated. Automation of latency scaling may be set in configuration settings of the system 10. If motion scaling is automated, then step 214 the controller 21a enables motion scaling by adjusting the input of the hand controllers 38a by adjusting the motion scaling factor. If the motion scaling is not automated, then at step 216, the controller 21a outputs a prompt on a graphical user interface (GUI) displayed on one of the screens 32 and 34. The prompt includes text or graphical elements recommending that motion scaling is enabled and may include a choice, e.g., YES or NO. The prompt may be dismissed or the user may select to enable motion scaling. If selected, then the controller 21a enables motion scaling by adjusting the input of the hand controllers 38a by adjusting the motion scaling factor.

Motion scaling may be calculated by the controller 21a using a factor, a multiplier or any other suitable function for translating user motion inputs at the handle controllers 38a and 38b into commanded motion of the robotic arms 40a-d. Thus, a magnitude of a motion vector of a movement of the handle controller may be multiplied by the scaling factor. To account for latency the motion scaling factor is adjusted in order to compensate for the delays introduced into either the imaging or the control pipeline.

In cases where abnormally high latency is detected, either in the imaging or control pipeline, the system 10 may be also configured to implement further measures to restore optimal functionality. In addition to adjusting the motion scaling factor, the system controller 21a may initiate recommended or automated responses involving partial or complete system reboots and/or software component restarts. The controller 21a evaluates the severity of the latency and determines whether a prompt should be displayed to the user on the graphical user interface (GUI). The prompt recommends a system restart to address the latency and asks for user confirmation.

When the detected latency exceeds a critical threshold, indicating the system 10 is no longer usable without significant risk or error, the controller can automatically initiate a partial or full system reboot. This automated response ensures system integrity is maintained while minimizing the impact on the procedure. A partial reboot might involve restarting specific software components like the video processing device or instrument control systems to address any isolated latency issues.

In cases where latency exceeds acceptable but non-critical levels, the controller displays a recommendation for the user to initiate a reboot of either the entire system or selected components. This prompt allows the user to confirm the appropriate corrective measure based on the current phase of the surgical procedure. The controller 21a will adjust the scaling factor and apply latency compensation until a suitable system restart can be performed, minimizing the disruption to the operation.

It will be understood that various modifications may be made to the embodiments disclosed herein. Therefore, the above description should not be construed as limiting, but merely as exemplifications of various embodiments. Those skilled in the art will envision other modifications within the scope and spirit of the claims appended thereto.

The invention may be described by reference to the following numbered paragraphs:-
1. A surgical robotic system comprising:
   a surgical robotic arm including an instrument and an instrument drive unit actuating the instrument;
   a surgeon console including a handle controller receiving user motion input to control at least one of the surgical robotic arm or the instrument using a motion scaling factor;
   a laparoscopic camera capturing a video feed; and
   a controller determining a latency attributable to at least one of the video feed or the user motion input and adjusting the motion scaling factor to compensate for the latency.
2. The surgical robotic system according to paragraph 1, wherein the controller determines the latency by determining imaging latency of the video feed.
3. The surgical robotic system according to paragraph 2, wherein the surgical robotic system further includes:
   a video processing device coupled to the laparoscopic camera, the video processing device receiving and processing the video feed; and
   a screen disposed at the surgeon console, the screen displaying the video feed.
4. The surgical robotic system according to paragraph 3, wherein the imaging latency includes a delay between capturing of the video feed by the laparoscopic camera and displaying the video feed on the screen.
5. The surgical robotic system according to paragraph 1, wherein the controller determines the latency by determining control latency of the user motion input.
6. The surgical robotic system according to paragraph 5, wherein the control latency includes a delay between receiving the user motion input and actuating at least one of the surgical robotic arm or the instrument.
7. The surgical robotic system according to paragraph 1, wherein the controller further compares the determined latency to a threshold latency and selects an adjusted motion scaling factor in response to the determined latency exceeding the threshold latency.
8. The surgical robotic system according to paragraph 7, wherein in response to the determined latency exceeding the threshold latency, the controller adjusts the motion scaling factor automatically.
9. The surgical robotic system according to paragraph 7, wherein in response to the determined latency exceeding the threshold latency, the controller outputs a prompt on a graphical user interface indicating the determined latency exceeds the threshold latency.
10. The surgical robotic system according to paragraph 9, wherein the controller activates latency compensation by adjusting the motion scaling factor in response to a user selection input to the prompt.
11. A method for controlling a surgical robotic system, the method comprising:
   receiving user motion input at a handle controller of a surgeon console for controlling at least one of a surgical robotic arm or an instrument using a motion scaling factor;
   capturing a video feed of a surgical site through a laparoscopic camera;
   determining a latency attributable to at least one of the video feed or the user motion input; and
   adjusting the motion scaling factor to compensate for the latency.
12. The method according to paragraph 11, wherein determining the latency further includes determining imaging latency of the video feed.
13. The method according to paragraph 12, further comprising
   receiving and processing the video feed at a video processing device coupled to the laparoscopic camera; and
   displaying the video feed at a screen disposed at the surgeon console.
14. The method according to paragraph 13, wherein the imaging latency includes a delay between capturing of the video feed by the laparoscopic camera and displaying the video feed on the screen.
15. The method according to paragraph 11, wherein determining the latency further includes determining control latency of the user motion input.
16. The method according to paragraph 15, wherein the control latency includes a delay between receiving the user motion input and actuating at least one of the surgical robotic arm or the instrument.
17. The method according to paragraph 11, further comprising:
   comparing the determined latency to a threshold latency; and
   selecting an adjusted motion scaling factor in response to the determined latency exceeding the threshold latency.
18. The method according to paragraph 17, further comprising:
   outputting a prompt on a graphical user interface indicating the determined latency exceeds the threshold latency in response to the determined latency exceeding the threshold latency the controller; and
   activating latency compensation by adjusting the motion scaling factor in response to a user selection input to the prompt.
19. A surgical robotic system comprising:
   a surgical robotic arm including an instrument and an instrument drive unit actuating the instrument;
   a storage device storing a baseline motion scaling factor range based on a latency calibration;
   a surgeon console including a handle controller receiving user motion input to control at least one of the surgical robotic arm or the instrument using a motion scaling factor selected from the baseline motion scaling factor range;
   a laparoscopic camera capturing a video feed; and
   a controller determining a latency attributable to at least one of the video feed or the user motion input and adjusting the motion scaling factor to compensate for the latency.
20. The surgical robotic system according to paragraph 19, wherein the controller compares the determined latency to a threshold latency and selects an adjusted motion scaling factor in response to the determined latency exceeding the threshold latency.

## Claims

1. A surgical robotic system (10) comprising:
a surgical robotic arm (40) including an instrument (50) and an instrument drive unit (52) actuating the instrument;
a surgeon console (30) including a handle controller (38a, 38b) receiving user motion input to control at least one of the surgical robotic arm or the instrument using a motion scaling factor;
a laparoscopic camera (51) capturing a video feed; and
a controller (21a) determining a latency attributable to at least one of the video feed or the user motion input and adjusting the motion scaling factor to compensate for the latency.

2. The surgical robotic system according to claim 1, wherein the controller determines the latency by determining imaging latency of the video feed.

3. The surgical robotic system according to any preceding claim, wherein the surgical robotic system further includes:
a video processing device (56) coupled to the laparoscopic camera, the video processing device receiving and processing the video feed; and
a screen (32, 34) disposed at the surgeon console, the screen displaying the video feed.

4. The surgical robotic system according to claim 3, wherein the imaging latency includes a delay between capturing of the video feed by the laparoscopic camera and displaying the video feed on the screen.

5. The surgical robotic system according to any preceding claim, wherein the controller determines the latency by determining control latency of the user motion input.

6. The surgical robotic system according to claim 5, wherein the control latency includes a delay between receiving the user motion input and actuating at least one of the surgical robotic arm or the instrument.

7. The surgical robotic system according to any preceding claim, wherein the controller further compares the determined latency to a threshold latency and selects an adjusted motion scaling factor in response to the determined latency exceeding the threshold latency.

8. The surgical robotic system according to claim 7, wherein in response to the determined latency exceeding the threshold latency, the controller adjusts the motion scaling factor automatically.

9. The surgical robotic system according to claim 7, wherein in response to the determined latency exceeding the threshold latency, the controller outputs a prompt on a graphical user interface indicating the determined latency exceeds the threshold latency.

10. The surgical robotic system according to claim 9, wherein the controller activates latency compensation by adjusting the motion scaling factor in response to a user selection input to the prompt.
